# EUROPEAN PATENT APPLICATION

(11) **EP 3 868 370 A1**
(43) Date of publication of application: **25.08.2021**
(21) Application number: 19872633.3
(22) Date of filing: 16.10.2019
(51) Int. Cl.: A61K 31/194, A61K 9/20, A61K 33/00, A61K 45/00, A61P 7/12, A61P 13/10, A61P 13/12

(54) **THERAPEUTIC OR PROPHYLACTIC AGENT FOR NOCTURIA**

(30) Priority: 17.10.2018 JP 2018195570
(71) Applicant: Nippon Chemiphar Co., Ltd., Tokyo 101-0032 (JP)
(72) Inventor: ARUGA Seiji, Suwa-shi, Nagano 392-0002 (JP); NISHIOKA Koichiro, Tokyo 101-0032 (JP); YAMASAKI Satomi, Tokyo 101-0032 (JP)
(74) Representative: Beckmann, Claus
(86) International application number: PCT/JP2019/040658
(87) International publication number: WO 2020/080399

(57) **Abstract**

There is provided a pharmaceutical composition which includes, as an active ingredient, a combination drug formulation of potassium citrate and sodium citrate hydrate or a sodium bicarbonate formulation. The prevention or treatment of nocturnal polyuria is achieved by administering the pharmaceutical composition.

## Description

### Technical Field

The present invention relates to use of citric acid, a pharmaceutically acceptable salt thereof or hydrates thereof or mixtures thereof, and sodium bicarbonate for treating or preventing nocturnal polyuria.

The present application claims priority based on Japanese Patent Application No. 2018-195570 filed in Japan on October 17, 2018, and the contents are incorporated herein by reference.

### Background Art

With the rapid aging of the population, urination-related complaints have recently increased. One of them is nocturnal polyuria. The term "nocturnal polyuria" refers to a condition in which the nocturnal urine output is increased, and a case in which the urine output during night-time sleep in elderly individuals exceeds 33% of the total daily urine output or a case in which the urine output during night-time sleep in young individuals exceeds 20% of the total daily urine output is defined as nocturnal polyuria (Patent Literatures 1 and 2).

Nocturnal polyuria is observed in 30 to 50% of the elderly individuals and is a major cause of a symptom called "nocturia", in which the elderly individuals need to urinate during their night-time sleep and have to get up at least once to urinate. In fact, nocturnal polyuria is found in 80% of patients with nocturia (Non Patent Literature 1).

As described above, nocturnal polyuria is the main cause of nocturia, and decreased secretion of nocturnal antidiuretic hormone: arginine vasopressin (AVP) in the elderly individuals is considered to be one of the causes of an increase in nocturnal urine output (Non Patent Literature 2). Accordingly, stimulation of the V2 receptor, which is an AVP receptor and exerts an antidiuretic effect, is expected to lead to improvement in nocturnal polyuria and to improvement in nocturia. In fact, desmopressin (dDAVP), i.e., the V2 receptor-selective agonist, has been reported to reduce nocturnal urine output and frequency of nocturnal urination (Non Patent Literatures 3 and 4).

However, the V2 receptor agonist theoretically promotes fluid retention and there is concern about hyponatremia. Therefore, it has been reported that when the V2 receptor agonist is administered to the elderly individuals, who account for the majority of patients with nocturnal polyuria and patients with nocturia, caution should be exercised, such as measurement of serum sodium level (Patent Literature 1).

### Citation List

### Patent Literature

Patent Literature 1: WO 2016/143200 A
Patent Literature 2: WO 2012/001469 A

Non Patent Literature

Non Patent Literature 1: J. Urol., 2011; 186: 1358-1363
Non Patent Literature 2: Drugs Aging, 1999; 15: 429-437
Non Patent Literature 3: J. Urol., 2013; 190: 958-964
Non Patent Literature 4: J. Urol., 2013; 190: 965-972

### Summary of Invention

### Technical Problem

One object of the present invention is to provide a pharmaceutical composition useful in treating or preventing nocturnal polyuria. One object of the present invention is to provide a pharmaceutical or food composition useful in reducing the frequency of nocturnal urination. One object of the present invention is to provide a pharmaceutical composition useful in treating or preventing nocturnal enuresis. One object of the present invention is to provide a pharmaceutical composition useful for use in combination with a V2 receptor agonist.

### Solution to Problem

The present inventors have conducted diligent studies to achieve the above objects, and found that citric acid, a pharmaceutically acceptable salt of citric acid, or hydrates thereof, or mixtures thereof, or sodium bicarbonate decreases the nocturnal urine output and the frequency of nocturnal urination, and completed the present invention.

In one aspect, the present invention provides a therapeutic or prophylactic agent for nocturnal polyuria which comprises citric acid, a pharmaceutically acceptable salt of citric acid, or hydrates thereof, or mixtures thereof, or sodium bicarbonate.

In one aspect, the present invention provides an agent for suppressing frequency of nocturnal urination which comprises citric acid, a pharmaceutically acceptable salt of citric acid, or hydrates thereof, or mixtures thereof, or sodium bicarbonate.

In one aspect, the present invention provides a therapeutic or prophylactic agent for nocturnal enuresis which comprises citric acid, a pharmaceutically acceptable salt of citric acid, or hydrates thereof, or mixtures thereof, or sodium bicarbonate.

In one aspect, the present invention provides use of a pharmaceutical composition comprising citric acid, a pharmaceutically acceptable salt of citric acid, or hydrates thereof, or mixtures thereof, or sodium bicarbonate in combination with a V2 receptor agonist.

### Advantageous Effects of Invention

The pharmaceutical composition or food composition provided by the present invention suppresses nocturnal polyuria and reduces the frequency of nocturnal urination.

### Description of Embodiments

The pharmaceutical composition provided by the present invention may comprise, as an active ingredient, citric acid, a pharmaceutically acceptable salt of citric acid, hydrates thereof, or mixtures thereof. Examples of pharmaceutically acceptable salts of citric acid include alkali metal salts of citric acid. Examples of alkali metal salts of citric acid include potassium citrate and sodium citrate, and mixtures thereof may be used. Potassium citrate and sodium citrate may be, for example, hydrates such as stable potassium citrate monohydrate (C₆H₅K₃O₇·H₂O) and sodium citrate dihydrate (C₆H₅Na₃O₇·2H₂O), respectively.

Examples of preferred active ingredients include anhydrous citric acid, sodium citrate, potassium citrate or a hydrate thereof, or mixtures thereof. For example, a mixture of potassium citrate monohydrate (C₆H₅K₃O₇·H₂O) and sodium citrate dihydrate (C₆H₅Na₃O₇·2H₂O); or a mixture of potassium citrate monohydrate (C₆H₅K₃O₇·H₂O), sodium citrate dihydrate (C₆H₅Na₃O₇·2H₂O), and anhydrous citric acid may be used.

In one embodiment, the mixing ratio of the number of moles of sodium salt of citric acid and the number of moles of potassium salt of citric acid in the mixture can be appropriately set by those skilled in the art, and is preferably in a range of 0.85 : 1.15 to 1.15 : 0.85, more preferably in a range of 0.90 : 1.10 to 1.10 : 0.90, more preferably in a range of 0.95 : 1.05 to 1.05 : 0.95, still more preferably in a range of 0.99 : 1.01 to 1.01 : 0.99, and particularly preferably 1 : 1.

In one embodiment, the mixing ratio of the number of moles of sodium citrate dihydrate (C₆H₅Na₃O₇·2H₂O) and the number of moles of potassium citrate monohydrate (C₆H₅K₃O₇·H₂O) in the mixture can be appropriately set by those skilled in the art, and is preferably in a range of 0.85 : 1.15 to 1.15 : 0.85, more preferably in a range of 0.90 : 1.10 to 1.10 : 0.90, more preferably in a range of 0.95 : 1.05 to 1.05 : 0.95, still more preferably in a range of 0.99 : 1.01 to 1.01 : 0.99, and particularly preferably 1 : 1.

In one embodiment, the mixing ratio of the number of moles of anhydrous citric acid, the number of moles of sodium salt of citric acid, and the number of moles of potassium salt of citric acid in the mixture can be appropriately set by those skilled in the art, and is preferably in a range of 1 : 1.7-2.3 : 1.7-2.3, more preferably in a range of 1 : 1.9-2.1 : 1.9-2.1, still more preferably in a range of 1 : 1.95-2.05 : 1.95-2.05, and particularly preferably 1 : 2 : 2.

In one embodiment, the mixing ratio of the number of moles of anhydrous citric acid, the number of moles of sodium citrate dihydrate (C₆H₅Na₃O₇·2H₂O), and the number of moles of potassium citrate monohydrate (C₆H₅K₃O₇·H₂O) in the mixture can be appropriately set by those skilled in the art, and is preferably in a range of 1 : 1.7-2.3 : 1.7-2.3, more preferably in a range of 1 : 1.9-2.1 : 1.9-2.1, still more preferably in a range of 1 : 1.95-2.05: 1.95-2.05, and particularly preferably 1 : 2 : 2.

In one embodiment, those skilled in the art can appropriately set the mixing ratio of potassium citrate monohydrate (C₆H₅K₃O₇·H₂O) and sodium citrate dihydrate (C₆H₅Na₃O₇·2H₂O). For example, the molar ratio of potassium citrate monohydrate to sodium citrate dihydrate may be in a range of 1 : 0.01 to 1 : 100. The mixing ratio may be about 1 : 1 in molar ratio.

In one embodiment, those skilled in the art can appropriately set the mixing ratio of potassium citrate monohydrate (C₆H₅K₃O₇·H₂O), sodium citrate dihydrate (C₆H₅Na₃O₇·2H₂O), and anhydrous citric acid in a mixture of potassium citrate monohydrate (C₆H₅K₃O₇·H₂O), sodium citrate dihydrate (C₆H₅Na₃O₇·2H₂O), and anhydrous citric acid. For example, as for the molar ratio of potassium citrate monohydrate to sodium citrate dihydrate and anhydrous citric acid, the molar ratio of potassium citrate monohydrate to sodium citrate dihydrate may be in a range of 1 : 0.01 to 1 : 100, and the molar ratio of potassium citrate monohydrate to anhydrous citric acid may be in a range of 1 : 0.01 to 1 : 100. The mixing ratio may be about 2 : 2 : 1 in molar ratio.

Further, other examples of preferred active ingredients include sodium citrate or a hydrate thereof. For example, sodium citrate dihydrate (C₆H₅Na₃O₇·2H₂O) may be used.

Further, other examples of preferred active ingredients include potassium citrate or a hydrate thereof. For example, potassium citrate monohydrate (C₆H₅K₃O₇·H₂O) may be used.

Further, as the active ingredient, sodium bicarbonate (baking soda) may be used instead of citric acid or a hydrate thereof, a pharmaceutically acceptable salt of citric acid or a hydrate thereof, or mixtures thereof.

In one embodiment, the active ingredient of the pharmaceutical composition provided by the present invention may include a mixture of citric acid or a hydrate thereof, sodium citrate or a hydrate thereof and potassium citrate or a hydrate thereof, or may be comprised only of a mixture of citric acid or a hydrate thereof, sodium citrate or a hydrate thereof and potassium citrate or a hydrate thereof.

In one embodiment, the active ingredient of the pharmaceutical composition provided by the present invention may include a mixture of sodium citrate or a hydrate thereof and potassium citrate or a hydrate thereof, or may be comprised only of a mixture of sodium citrate or a hydrate thereof and potassium citrate or a hydrate thereof.

In one embodiment, the active ingredient of the pharmaceutical composition provided by the present invention includes citric acid, a pharmaceutically acceptable salt of citric acid or hydrates thereof or mixtures thereof, excluding ferrous citrate, ferric citrate and hydrates thereof (e.g., ferric citrate hydrate).

In the present specification, when referring to the weight of citric acid or a hydrate thereof, a pharmaceutically acceptable salt of citric acid or a hydrate thereof, or mixtures thereof (e.g., potassium citrate monohydrate (C₆H ₅K₃O₇·H₂O) and sodium citrate dihydrate (C₆H₅Na₃O₇·2H₂O)), the weight may be dry weight.

The pharmaceutical composition provided by the present invention is useful in reducing the nocturnal urine production and in decreasing the frequency of nocturnal urination.

Therefore, in one embodiment, the present invention provides a pharmaceutical composition for reducing nocturnal urine production. In one embodiment, the present invention provides a pharmaceutical composition for treating or preventing nocturnal polyuria. In one embodiment, the present invention provides a pharmaceutical composition for suppressing frequency of nocturnal urination. In one embodiment, the present invention provides a pharmaceutical composition for treating or preventing nocturia. In one embodiment, the present invention provides a pharmaceutical composition for reducing urge to urinate at night. In one embodiment, the present invention provides a pharmaceutical composition for treating or preventing nocturia due to nocturnal polyuria. Alternatively, in one embodiment, the present invention provides a pharmaceutical composition for treating nocturia due to nocturnal polyuria in men.

In the present specification, the term "nocturnal polyuria" refers to a state in which the nocturnal urine output is increased; for example, in the elderly individuals, the urine output during night-time sleep exceeds 33% of the total daily urine output, and in young individuals, the urine output during night-time sleep exceeds 20% of the total daily urine output. Nocturia is a symptom that the individuals have to wake up at least once to urinate during sleep.

The decreased frequency of nocturnal urination and the reduced nocturnal urine production may also be useful in treating or preventing nocturnal enuresis.

Thus, the present invention provides, in one embodiment, a pharmaceutical composition for treating or preventing nocturnal enuresis.

In one embodiment, the pharmaceutical composition provided by the present invention does not affect the daily urine output.

In one embodiment, the pharmaceutical composition provided by the present invention does not affect the frequency of urination per day.

The V2 receptor agonist is known to be effective for nocturnal polyuria and nocturia. Thus, the pharmaceutical composition provided by the present invention may be used in combination with the V2 receptor agonist (e.g., desmopressin or a pharmaceutically acceptable salt thereof, or hydrates thereof (e.g., desmopressin acetate hydrate)).

In one embodiment, the present invention provides use of the pharmaceutical composition provided by the present invention in combination with the V2 receptor agonist. The combination use can reduce the nocturnal urine output and/or decrease the frequency of nocturnal urination. Thus, in one embodiment, there is provided use of the pharmaceutical composition provided by the present invention in combination with the V2 receptor agonist (e.g., desmopressin or a pharmaceutically acceptable salt thereof, or hydrates thereof (e.g., desmopressin acetate hydrate)) in order to treat or prevent nocturnal polyuria and/or nocturia.

Since the V2 receptor agonist has an antidiuretic effect, it is used in the treatment of nocturnal enuresis (e.g., nocturnal enuresis associated with decreased urine osmolality or urine specific gravity) and central diabetes insipidus. Since the pharmaceutical composition provided by the present invention does not interfere with the antidiuretic effect of the V2 receptor agonist, but rather has a different action mechanism from the V2 receptor agonist, and the pharmaceutical composition can cooperate with the V2 receptor agonist. Accordingly, in one embodiment, the present invention provides use of the pharmaceutical composition provided by the present invention in combination with the V2 receptor agonist (e.g., desmopressin or a pharmaceutically acceptable salt thereof, or hydrates thereof (e.g., desmopressin acetate hydrate)) in order to treat or prevent nocturnal enuresis (e.g., nocturnal enuresis associated with decreased urine osmolality or urine specific gravity) and central diabetes insipidus.

Hyponatremia has been reported as a side effect of V2 receptor agonist, and the pharmaceutical composition provided by the present invention may include sodium citrate or a hydrate thereof. Therefore, the combination use thereof can suppress the side effect of V2 receptor agonist and enhance the antidiuretic effect exerted by the V2 receptor agonist.

Thus, in one embodiment, the present invention provides a pharmaceutical composition comprising sodium citrate or a hydrate thereof (e.g., a pharmaceutical composition comprising sodium citrate dihydrate; a pharmaceutical composition comprising potassium citrate or a hydrate thereof, and sodium citrate or a hydrate thereof; or a pharmaceutical composition comprising a potassium citrate monohydrate and sodium citrate dihydrate) in combination with a V2 receptor agonist (e.g., desmopressin or a pharmaceutically acceptable salt thereof, or hydrates thereof (e.g., desmopressin acetate hydrate). In one embodiment, the present invention provides a pharmaceutical composition comprising sodium citrate or a hydrate thereof (e.g., a pharmaceutical composition comprising sodium citrate dihydrate; a pharmaceutical composition comprising potassium citrate or a hydrate thereof, and sodium citrate or a hydrate thereof; or a pharmaceutical composition comprising potassium citrate monohydrate and sodium citrate dihydrate) in combination with a V2 receptor agonist (e.g., desmopressin or a pharmaceutically acceptable salt thereof, or hydrates thereof (e.g., desmopressin acetate hydrate)), in order to treat or prevent nocturnal polyuria and/or nocturia (e.g., nocturia due to nocturnal polyuria (e.g., idiopathic nocturnal polyuria)).

In one embodiment, there is provided use of a pharmaceutical composition comprising sodium citrate or a hydrate thereof (e.g., a pharmaceutical composition comprising sodium citrate dihydrate; a pharmaceutical composition comprising potassium citrate or a hydrate thereof, and sodium citrate or a hydrate thereof, or a pharmaceutical composition comprising potassium citrate monohydrate and sodium citrate dihydrate) in combination with a V2 receptor agonist (e.g., desmopressin or a pharmaceutically acceptable salt thereof, or hydrates thereof (e.g., desmopressin acetate hydrate)), in order to treat or prevent nocturnal enuresis (e.g., nocturnal enuresis associated with decreased urine osmolality or urine specific gravity) and central diabetes insipidus.

The combination use may be achieved either by administering the pharmaceutical composition provided by the present invention and a pharmaceutical composition comprising the V2 receptor agonist (e.g., desmopressin or a pharmaceutically acceptable salt thereof, or hydrates thereof (e.g., desmopressin acetate hydrate) as a single composition or by administering these ingredients as separate compositions.

In one embodiment, the present invention provides a pharmaceutical composition which comprises, as active ingredients, i) citric acid, a pharmaceutically acceptable salt of citric acid, or hydrates thereof, or mixtures thereof (e.g., a mixture of potassium citrate monohydrate (C₆H₅K₃O₇·H₂O) and sodium citrate dihydrate (C₆H₅Na₃O₇·2H₂O), or a mixture of potassium citrate monohydrate (C₆H₅K₃O₇·H₂O), sodium citrate dihydrate (C₆H₅Na₃O₇·2H₂O), and anhydrous citric acid); and ii) a V2 receptor agonist (e.g., desmopressin or a pharmaceutically acceptable salt thereof, or hydrates thereof (e.g., desmopressin acetate hydrate)).

In one embodiment, the present invention provides use of sodium bicarbonate (baking soda) in combination with a V2 receptor agonist (e.g., desmopressin or a pharmaceutically acceptable salt thereof, or hydrates thereof (e.g., desmopressin acetate hydrate). In one embodiment, the present invention provides use of sodium bicarbonate (baking soda) in combination with a V2 receptor agonist (e.g., desmopressin or a pharmaceutically acceptable salt thereof, or hydrates thereof (e.g., desmopressin acetate hydrate), for treating or preventing i) nocturnal polyuria and/or nocturia (e.g., nocturia due to nocturnal polyuria (e.g., idiopathic nocturnal polyuria)); or ii) nocturnal enuresis (e.g., nocturnal enuresis associated with decreased urine osmolality or urine specific gravity) and central diabetes insipidus.

In one embodiment, the present invention provides a pharmaceutical composition comprising: i) sodium bicarbonate (baking soda); and ii) a V2 receptor agonist (e.g., desmopressin or a pharmaceutically acceptable salt thereof, or hydrates thereof (e.g., desmopressin acetate hydrate)) as an active ingredient.

The subject of administration of the pharmaceutical composition provided by the present invention may be a mammal (e.g., a human). The subject is not particularly limited and may be appropriately selected by those skilled in the art.

Since nocturnal polyuria and increased frequency of nocturnal urination are commonly seen in elderly individuals, in one embodiment, the pharmaceutical composition provided by the present invention is administered to an elderly individual (e.g., 60 years old or older, 65 years old or older, 70 years old or older, 75 years old or older, 65 years old or older and under 75).

In one embodiment, the pharmaceutical composition provided by the present invention is administered to a human 40 years old or older, 50 years old or older, or 40 years old or older and under 60.

In one embodiment, the pharmaceutical composition provided by the present invention is administered to a male human.

In one embodiment, the pharmaceutical composition provided by the present invention is administered to a male human who has nocturia due to nocturnal polyuria.

Further, in chronic kidney disease, urination disorders such as polyuria and nocturnal polyuria is observed from a relatively early stage (e.g., a human whose CKD stage is G3a or G3b). Accordingly, in one embodiment, the pharmaceutical composition provided by the present invention is administered to a patient with chronic kidney disease (e.g., a human whose CKD stage is G3a or G3b).

Furthermore, nocturnal enuresis is a problem in young individuals. Accordingly, in one embodiment, the pharmaceutical composition provided by the present invention is administered to a young individual (e.g., an infant (e.g., a child 3 years old or older and under 6), a child (e.g., a child 6 years old or older), a child aged between 6 and 12, and a child aged between 6 and 15).

In one embodiment, the subject of administration of the pharmaceutical composition provided by the present invention does not suffer from gout.

In one embodiment, the subject of administration of the pharmaceutical composition provided by the present invention does not suffer from hyperuricemia.

In one embodiment, the subject of administration of the pharmaceutical composition provided by the present invention is not a subject (e.g., a human) in need of improvement of acidic urine in gout and hyperuricemia.

In one embodiment, the subject of administration of the pharmaceutical composition provided by the present invention is not a subject (e.g., a human) in need of improvement of acidosis.

In one embodiment, the pharmaceutical composition provided by the present invention is not used in combination with a uric acid lowering agent (e.g., a uric acid excretion promoter or a uric acid production inhibitor).

The pharmaceutical composition provided by the present invention is orally or parenterally administered to a human or another mammal, and examples of parenteral administration include intravenous administration, subcutaneous administration, intramuscular administration, intraarticular administration, and transmucosal administration, transdermal administration, nasal administration, rectal administration, intrathecal administration, intraperitoneal administration, and local administration. The frequency of administration can be appropriately set by those skilled in the art, and may be, for example, once per day, twice per day, or three times per day.

The pharmaceutical composition provided by the present invention may be prepared by using citric acid, a pharmaceutically acceptable salt of citric acid, or hydrates thereof, or mixtures thereof, or sodium bicarbonate as it is, or may be prepared by mixing citric acid, a pharmaceutically acceptable salt of citric acid, or hydrates thereof, or mixtures thereof, or sodium bicarbonate with a pharmaceutically acceptable carrier, such as an excipient (e.g., lactose, D-mannitol, crystalline cellulose, or glucose), a binder (e.g., hydroxypropylcellulose (HPC), gelatin or polyvinylpyrrolidone (PVP)), a lubricant (e.g., magnesium stearate or talc), a disintegrant (e.g., starch or carboxymethylcellulose calcium (CMC-Ca)), a diluent (e.g., water for injection or physiological saline), and, if necessary, other additives (e.g., a pH adjuster, a surfactant, a solubilizing agent, a preservative, an emulsifier, an isotonic agent, or a stabilizer), and may be a formulation such as a tablet, a capsule, a suspension, an injection, or a suppository. For example, in order to make tablets, citric acid, a pharmaceutically acceptable salt thereof, or hydrates thereof, or mixtures thereof, or sodium bicarbonate may be mixed with an excipient (e.g., lactose, D-mannitol, crystalline cellulose or glucose), a disintegrant (e.g., starch or carboxymethylcellulose calcium (CMC)-Ca)), a binder (e.g., hydroxypropylcellulose (HPC), gelatin, or polyvinylpyrrolidone (PVP)), a lubricant (e.g., magnesium stearate or talc), and the like for formulation.

The tablets according to the present invention will be described in more detail below.

In one embodiment, the pharmaceutical composition provided by the present invention is a tablet. The tablet provided by the present invention may comprise citric acid, a pharmaceutically acceptable salt of citric acid, or hydrates thereof, or mixtures thereof, or sodium bicarbonate. More specifically, the tablet may comprise, for example, an active ingredient such as citric acid or a hydrate thereof; potassium citrate or a hydrate thereof; sodium citrate or a hydrate thereof; a mixture of potassium citrate or a hydrate thereof and sodium citrate or a hydrate thereof; a mixture of potassium citrate monohydrate or sodium citrate dihydrate; a mixture of citric acid or a hydrate thereof, potassium citrate or a hydrate thereof, and sodium citrate or a hydrate thereof; a mixture of citric acid or a hydrate thereof, potassium citrate monohydrate and sodium citrate dihydrate; or sodium bicarbonate, as well as pharmaceutically acceptable additives commonly used in the pharmaceutical field. Examples of such additives include excipients, binders, disintegrants, fluidizers, flavoring agents, lubricants, pH adjusters, surfactants, stabilizers, and fragrances.

The content of citric acid or a hydrate thereof; potassium citrate or a hydrate thereof; sodium citrate or a hydrate thereof; a mixture of potassium citrate or a hydrate thereof, and sodium citrate or a hydrate thereof; a mixture of potassium citrate monohydrate and sodium citrate dihydrate; a mixture of citric acid or a hydrate thereof, potassium citrate or a hydrate thereof, and sodium citrate or a hydrate thereof; a mixture of citric acid or a hydrate thereof, potassium citrate monohydrate and sodium citrate dihydrate; or sodium bicarbonate in the tablet provided by the present invention may be in a range of 10 to 95% by weight, preferably in a range of 30 to 90% by weight, more preferably in a range of 60 to 85% by weight relative to the tablet, or may be in a range of 10 mg to 1 g per tablet.

Examples of excipients that can be used in the tablet provided by the present invention include sugars such as lactose (e.g., lactose hydrate and anhydrous lactose), glucose, sucrose, fructose, and maltose; sugar alcohols such as erythritol, sorbitol, maltitol, xylitol, and D-mannitol; starch (e.g., corn starch, potato starch, rice starch, and wheat starch), crystalline cellulose, magnesium aluminometasilicate, anhydrous calcium phosphate, precipitated calcium carbonate, calcium silicate, calcium lactate, and ethyl cellulose. Particularly, crystalline cellulose is preferable.

The content of the excipient in the tablet provided by the present invention may be in a rage of 1 to 95% by weight, preferably in a rage of 1 to 80% by weight, more preferably in a rage of 3 to 80% by weight, and still more preferably in a rage of 3 to 20% by weight relative to the tablet.

Examples of binders that can be used in the tablet provided by the present invention include
hydroxypropylcellulose, hydroxypropylmethylcellulose, polyvinylpyrrolidone, dextrin, methylcellulose, polyvinyl alcohol, sodium alginate, aminoalkyl methacrylate copolymer, polyethylene glycol, pregelatinized starch (e.g., partially pregelatinized starch), agar, and gelatin. Particularly, hydroxypropyl cellulose is preferable.

The content of the binder in the tablet provided by the present invention may be in a rage of 0.1 to 30% by weight, preferably in a rage of 0.1 to 10% by weight, and more preferably in a rage o 0.3 to 3% by weight relative to the tablet.

Examples of disintegrants that can be used in the tablet provided by the present invention include croscarmellose sodium, carmellose calcium, sodium carboxymethyl starch, low substituted
hydroxypropylcellulose, crospovidone, starch (e.g., wheat starch, corn starch, or partially pregelatinized starch), and carmellose. Particularly, partially pregelatinized starch is preferable.

The content of the disintegrant in the tablet provided by the present invention may be in a rage of 0.3 to 20% by weight, preferably in a rage of 1 to 10% by weight, and more preferably in a rage of 3 to 10% by weight relative to the tablet.

Examples of fluidizing agents that can be used in the tablet provided by the present invention include light anhydrous silicic acid, talc, and magnesium aluminometasilicate.

The content of the fluidizing agent in the tablet provided by the present invention may be in a range of 0.03 to 3% by weight, preferably in a range of 0.1 to 3% by weight, and more preferably in a range of 0.3 to 3% by weight relative to the tablet.

Examples of flavoring agents that can be used in the tablet provided by the present invention include acidulants such as malic acid, acetic acid, tartaric acid, fumaric acid, ascorbic acid (provided that the flavoring agents do not include citric acid or an alkali metal salt thereof, or sodium bicarbonate, i.e., the active ingredient of the present invention), and sweeteners such as sodium saccharin, dipotassium glycyrrhizinate, aspartame (registered trademark), stevia, thaumatin, and sucralose.

The content of the flavoring agent in the tablet provided by the present invention may be in a range of 0.03 to 3% by weight, preferably in a range of 0.1 to 3% by weight, and more preferably in a range of 0.3 to 3% by weight relative to the tablet.

Examples of lubricants that can be used in the tablet provided by the present invention include magnesium stearate, calcium stearate, talc, light anhydrous silicic acid, sucrose fatty acid ester, carnauba wax, macrogol, and sodium stearyl fumarate. Particularly, magnesium stearate is preferable.

The content of the lubricant in the tablet provided by the present invention may be in a range of 0.1 to 30% by weight, preferably in a range of 0.3 to 10% by weight, more preferably in a range of 1 to 3% by weight relative to the tablet.

Examples of pH adjusters that can be used in the tablet provided by the present invention include phosphates (e.g., sodium dihydrogen phosphate and potassium dihydrogen phosphate), carbonates (e.g., magnesium carbonate and sodium carbonate), tartrates, fumarates, acetates, and amino acid salts (provided that the pH adjusters do not include citric acid or an alkali metal salt thereof, or sodium bicarbonate, i.e., the active ingredient of the present invention).

The content of the pH adjuster in the tablet provided by the present invention may be in a range of 0.1 to 30% by weight, preferably in a range of 0.3 to 10% by weight, and more preferably in a range of 1 to 5% by weight relative to the tablet.

Examples of surfactants that can be used in the tablets provided by the present invention include sodium lauryl sulfate, polysorbate, sucrose fatty acid ester, polyoxyethylene hydrogenated castor oil, polyoxyl stearate, macrogol, and poloxamer.

The content of the surfactant in the tablet provided by the present invention may be in a range of 0.01 to 3% by weight, preferably in a range of 0.03 to 1% by weight, and more preferably in a range of 0.03 to 0.5% by weight relative to the tablet.

Examples of stabilizers that can be used in the tablet provided by the present invention include malic acid, acetic acid, tartaric acid, maleic acid, ascorbic acid, sodium edetate, and tocopherol.

In one embodiment, when the active ingredient in the tablet provided by the present invention is an alkali metal salt of citric acid (e.g., a mixture of potassium citrate or a hydrate thereof and sodium citrate or a hydrate thereof; or a mixture of potassium citrate monohydrate and sodium citrate dihydrate), the tablet provided by the present invention may contain anhydrous citric acid as a stabilizer.

The content of the stabilizer in the tablet provided by the present invention may be in a range of 0.01 to 30% by weight, preferably in a range of 0.1 to 30% by weight, and more preferably in a range of 1 to 20% by weight relative to the tablet.

Examples of fragrances that can be used in the tablet provided by the present invention include citrus fragrances such as lemon, orange, grapefruit, peppermint, spearmint, and menthol, and these fragrances may be contained in an appropriate amount per tablet (for example, in a range of 0.01 to 1% by weight, and more preferably in a range of 0.01 to 0.1% by weight per tablet).

In the tablet provided by the present invention, the total content of the active ingredient, i.e., citric acid or an alkali metal salt thereof or sodium bicarbonate, and the pharmaceutically acceptable additives does not exceed 100% by weight relative to the tablet.

The tablet provided by the present invention may be an uncoated tablet comprising the above-described ingredients that is not subjected to coating treatment, or may be a film-coated tablet that is subjected to coating treatment. The content of the coating layer can be appropriately set by those skilled in the art, and may be, for example, in a range of 0.1 to 10% by weight relative to the uncoated tablet. The coating layer may appropriately contain a plasticizer, a colorant, a brightening agent, and the like, in addition to the coating base.

Examples of coating bases that can be used in the tablet provided by the present invention include hydroxypropylcellulose, hydroxypropylmethylcellulose, ethyl cellulose, cellulose acetate phthalate, methacrylic acid copolymer, and polyvinylpyrrolidone. Particularly, hydroxypropylmethylcellulose is preferable. The content of the coating base in the tablet provided by the present invention may be in a range of 0.01 to 10% by weight, and preferably in a range of 0.3 to 3% by weight relative to the tablet.

Examples of coating plasticizers that can be used in the tablet provided by the present invention include triethyl citrate, medium chain triglyceride, triacetin, glycerin, propylene glycol, and polyethylene glycol (e.g., macrogol 6000). Particularly, macrogol 6000 is preferable. The content of the coating plasticizer in the tablet provided by the present invention may be in a range of 0.01 to 1% by weight, and preferably in a range of 0.03 to 3% by weight relative to the tablet.

Examples of coating colorants that can be used in the tablet provided by the present invention include titanium oxide, yellow iron sesquioxide, iron sesquioxide, black iron oxide, FD & C BLUE No. 2, and FD & C BLUE 2 aluminum lake. The content of the coating colorant in the tablet provided by the present invention may be in a range of 0.01 to 1% by weight, and preferably in a range of 0.03 to 3% by weight relative to the tablet.

Examples of coating brighteners that can be used in the tablet provided by the present invention include carnauba wax. The content of the coating brightener in the tablet provided by the present invention may be in a range of 0.0001 to 0.1% by weight, and preferably in a range of 0.001 to 0.01% by weight relative to the tablet.

The pharmaceutical composition provided by the present invention can be produced by a method known in the pharmaceutical field. For example, in the case of making tablets, the production method comprises: mixing an active ingredient; citric acid, a pharmaceutically acceptable salt of citric acid, or hydrates thereof, or mixtures thereof, or sodium bicarbonate (more specifically, for example, potassium citrate or a hydrate thereof; sodium citrate or a hydrate thereof; a mixture of potassium citrate monohydrate and sodium citrate dihydrate; or sodium bicarbonate) with an additive; granulating the mixture; and tableting and/or coating the granulated product.

The mixing step may comprise mixing the active ingredient with an additive such as an excipient, a stabilizer, a disintegrant and/or a binder. Further, the step may include mixing the mixture containing the active ingredient and the additive with a lubricant, a flavoring agent and/or a fragrance before the tableting step. Mixing can be performed using a V-type mixer, a W-type mixer, a container mixer, a tumbler mixer, a stirring mixer, or the like.

The granulation step can be performed by a granulation method known in the pharmaceutical field. Examples of granulation methods include a dry granulation method, a wet granulation method, and a fluidized-bed granulation method.

In one embodiment, the mixture obtained in the mixing step and the granulated product obtained in the granulation step are appropriately pulverized and/or sieved to form a mixture or granulated product having a desired particle size. The pulverization can be performed by a pulverizer known in the pharmaceutical field such as a ball mill, a jet mill, or a hammer mill. The sieving can be performed using a 16 mesh sieve (opening of 1000 µm) to 32 mesh sieve (opening of 500 µm) or the like.

The tableting step can be performed by a tableting method known in the pharmaceutical field. Examples of tableting methods include a direct compression tableting method, a dry tableting method, a wet tableting method, and an external lubrication tableting method. For example, a tableting machine known in the pharmaceutical field, such as a single punch tableting machine or a rotary tableting machine, can be used to tablet the mixture or granulated product obtained in the above step. When the single punch tableting machine, the rotary tableting machine, or the like is used, a tableting pressure of 1 kN to 30 kN can be adopted.

The coating step can be performed by a method known in the pharmaceutical field. For example, the step can be performed by spray-coating the outside of the uncoated tablet with a coating liquid containing a coating base and a plasticizer, a colorant, a brightening agent, and the like as appropriate.

In one embodiment, the tablet provided by the present invention can be produced by mixing the active ingredient with an excipient (e.g., lactose, D-mannitol, crystalline cellulose and/or glucose), a binder (e.g., hydroxypropylcellulose (HPC)), gelatin and/or polyvinylpyrrolidone (PVP)), a stabilizer, a disintegrant (e.g., starch (e.g., partially pregelatinized starch) and/or carboxymethylcellulose calcium (CMC-Ca)) and a lubricant (e.g., magnesium stearate) and tableting the mixture to form an uncoated tablet; and forming a coating layer containing a coating base (e.g., hydroxypropylcellulose, hydroxypropylmethylcellulose and/or PVP), a plasticizer (e.g., triethyl citrate and/or macrogol 6000), a colorant (e.g., iron sesquioxide and/or titanium oxide), and a brightening agent (e.g., carnauba wax) on the outside of the uncoated tablet.

In one embodiment, the hardness of the resulting tablet may be in a range of 10 to 200 N, and preferably in a range of 30 to 150 N.

The amount of the active ingredient in the pharmaceutical composition provided by the present invention can be appropriately set.

In one embodiment, among the active ingredients in the pharmaceutical composition provided by the present invention, the amount of an alkaline agent such as an alkali metal salt of citric acid or sodium bicarbonate may be set to a value in which acidic urine in gout and hyperuricemia is improved by administering the alkaline agent to a human. For example, the amount may be set to the daily dose approved in Japan for improving acidic urine in gout and hyperuricemia (e.g., when the alkalizing agent is a citric acid formulation: two tablets each containing 231.5 mg of potassium citrate (C₆H₅K₃O₇·H₂O) and 195.0 mg of sodium citrate hydrate (C₆H₅Na₃O₇·2H₂O) are orally administered three times per day, when the alkalizing agent is sodium bicarbonate: 3 to 5 g of sodium bicarbonate is orally administered per day).

In one embodiment, the pharmaceutical composition provided by the present invention is a tablet, and may comprise potassium citrate monohydrate or sodium citrate dihydrate as an alkalizing agent in an amount of 10 mg to 1 g, preferably in an amount of 100 mg to 500 mg, more preferably in an amount of 400 mg to 500 mg per tablet.

In one embodiment, the pharmaceutical composition provided by the present invention is a tablet, and may comprise potassium citrate monohydrate and sodium citrate dihydrate, respectively, in an amount of 10 mg to 300 mg for a total of 20 mg to 600 mg, preferably in an amount of 150 to 250 mg for a total of 400 to 500 mg, more preferably in an amount of 190 to 240 mg for a total of 400 to 450 mg per tablet.

In one embodiment, the pharmaceutical composition provided by the present invention is a tablet, and may comprise potassium citrate monohydrate or sodium citrate dihydrate as an alkalizing agent in an amount of 10 mg to 1 g, preferably in an amount of 100 mg to 500 mg, more preferably in an amount of 400 mg to 500 mg per tablet, and may comprise anhydrous citric acid in an amount of 10 mg to 500 mg, preferably in an amount of 10 mg to 100 mg, more preferably in an amount of 50 mg to 100 mg per tablet.

In one embodiment, the pharmaceutical composition provided by the present invention is a tablet, and may comprise potassium citrate monohydrate and sodium citrate dihydrate, respectively, in an amount of 10 mg to 300 mg for a total of 20 mg to 600 mg, preferably in an amount of 150 to 250 mg for a total of 400 to 500 mg, more preferably in an amount of 190 to 240 mg for a total of 400 to 450 mg per tablet, and may comprise anhydrous citric acid in an amount of 10 mg to 500 mg, preferably in an amount of 10 mg to 100 mg, more preferably in an amount of 50 mg to 100 mg.

In one embodiment, the pharmaceutical composition provided by the present invention is a tablet, and may comprise sodium bicarbonate as an alkalizing agent in an amount of 10 mg to 1 g, preferably in an amount of 100 mg to 500 mg per tablet.

In one embodiment, the tablet of the pharmaceutical compositions provided by the present invention does not comprise gelatin.

In one embodiment, the tablet of the pharmaceutical composition provided by the present invention comprises gelatin, D-mannitol, anhydrous citric acid, and desmopressin acetate hydrate, and is a tablet, excluding an orally disintegrating tablet containing only desmopressin acetate hydrate as an active ingredient.

In one embodiment, the pharmaceutical composition provided by the present invention is a tablet, and may comprise 231.5 mg of potassium citrate monohydrate and 195.0 mg of sodium citrate dihydrate as active ingredients, and may comprise anhydrous citric acid, crystalline cellulose, partially pregelatinized starch, hydroxypropylcellulose, magnesium stearate, hypromellose, macrogol 6000, titanium oxide, and carnauba wax as additives. In this embodiment, the tablet provided by the present invention may comprise 72.5 mg of anhydrous citric acid as an additive.

In one embodiment, a tablet comprising 231.5 mg of potassium citrate monohydrate and 195.0 mg of sodium citrate dihydrate may be used as one dosage unit.

In the present specification, the "dosage unit" represents a unit of the formulation, and the "one dosage unit" represents the minimum unit of the formulation. Thus, for example, in the case of tablets, the dosage unit is each tablet and one dosage unit represents one tablet. In the case of an injection, the dosage unit is an injection placed in a sealed container such as an ampoule or a vial, and one administration unit represents an injection in a hermetically sealed container such as an ampoule or a vial.

When the pharmaceutical composition provided by the present invention is administered to a human or another mammal, one or more of the above-described dosage units may be administered at a time, and the one dosage unit may be administered in divided doses.

The administered dose of an active ingredient, which is citric acid, a pharmaceutically acceptable salt of citric acid, or hydrates thereof, or mixtures thereof, or sodium bicarbonate, is appropriately determined according to the type of the active ingredient, the administration method, the age, weight, sex, and symptoms of the subject to be administered, susceptibility to drugs, and the like. The administered dose may be adjusted according to the situation of symptom improvement.

In one embodiment, when the active ingredient; a mixture of potassium citrate monohydrate and sodium citrate dihydrate or sodium bicarbonate is orally administered to a human, the daily dose approved in Japan for improving acidic urine in gout and hyperuricemia (e.g., when the active ingredient is a citric acid formulation: two tablets each containing 231.5 mg of potassium citrate (C₆H₅K₃O₇·H₂O) and 195.0 mg of sodium citrate hydrate (C₆H₅Na₃O₇·2H₂O) are orally administered three times per day, when the active ingredient is sodium bicarbonate: 3 to 5 g of sodium bicarbonate is orally administered per day) may be used as the daily dose.

In one embodiment, when the active ingredient; a mixture of potassium citrate monohydrate and sodium citrate dihydrate is orally administered to a human, each of potassium citrate monohydrate and sodium citrate dihydrate may be administered in a dose of 0.1 to 5 g/day for a total of 0.2 to 10 g/day, 0.1 to 3 g/day for a total of 0.2 to 6 g/day, 0.5 to 3 g/day for a total of 1 to 6 g/day, preferably 0.5 to 1.5 g/day for a total of 1 to 3 g/day, 1 to 1.5 g/day for a total of 2 to 3 g/day, or 0.5 to 1 g/day for a total of 1 to 2 g/day, and may be administered daily in 1 to 5 divided doses, preferably 3 divided doses.

In one embodiment, when the active ingredient; potassium citrate monohydrate or sodium citrate dihydrate is orally administered to a human, it may be administered in a dose of 1 to 10 g/day, 1 to 6 g/day, 2 to 5.5 g/day, 1 to 3 g/day, 2 to 3 g/day, or 1 to 1.5 g/day, and may be administered daily in 1 to 5 divided doses, preferably 3 divided doses.

In one embodiment, when the active ingredient; a mixture of anhydrous citric acid, potassium citrate monohydrate and sodium citrate dihydrate is orally administered to a human, anhydrous citric acid is administered in a dose of 0.1 to 2 g/day, preferably 0.1 to 1 g/day, more preferably 0.3 to 0.6g/day, and each of potassium citrate monohydrate and sodium citrate dihydrate may be administered in a dose of 0.1 to 5 g/day for a total of 0.2 to 10 g/day, 0.1 to 3 g/day for a total of 0.2 to 6 g/day, 0.5 to 3 g/day for a total of 1 to 6 g/day, preferably 0.5 to 1.5 g/day for a total of 1 to 3 g/day, 1 to 1.5 g/day for a total of 2 to 3 g/day, or 0.5 to 1 g/day for a total of 1 to 2 g/day, or may be administered daily in 1 to 5 divided doses, preferably 3 divided doses.

In one embodiment, when the active ingredient; anhydrous citric acid is orally administered to a human, it may be administered in a dose of 0.1 to 2 g/day, preferably 0.1 to 1 g/day, more preferably 0.3 to 0.6 g/day, and may be administered daily in 1 to 5 divided doses, preferably 3 divided doses.

In one embodiment, when the active ingredient; sodium bicarbonate is orally administered to a human, it may be administered in a dose of 1 to 6g/day, preferably 1 to 3 g/day, or 3 to 5g/day, and may be administered daily in 1 to 5 divided doses, preferably 3 divided doses.

In one embodiment, the administered dose of the active ingredient may be set such that human urine (e.g., early-morning urine) ranges from pH 6.0 to pH 7.5, from pH 6.0 to pH 7.2, or from pH 6.2 to pH 7.0 by oral administration of the active ingredient.

In one embodiment, the administered dose of the active ingredient may be set such that, after 7 days of oral administration of the active ingredient, human urine (e.g., early-morning urine) ranges from H 6.0 to pH 7.5, from pH 6.0 to pH 7.2, or from pH 6.2 to pH 7.0.

The administration period of the pharmaceutical composition provided by the present invention can be appropriately set to, for example, 5 days, 1 week, 2 weeks, 1 month, 5 days or more and 10 days or less, 1 week or more and 2 weeks or less, 1 week or more and 1 or less, 5 days or more, 1 week or more, 2 weeks or more, or 1 month or more.

In one embodiment, the efficacy of the pharmaceutical composition provided by the present invention develops early, and the drug effect is achieved by administration for 5 days or 1 week (e.g., administration three times per day for 5 days or administration for 1 week).

Further, examples of the embodiments provided by the present invention include the following:
(1-1) Use of citric acid or a pharmaceutically acceptable salt, or hydrates thereof, or sodium bicarbonate for producing a pharmaceutical composition for treating or preventing nocturnal polyuria;
(1-2) Use of citric acid or a pharmaceutically acceptable salt, or hydrates thereof, or sodium bicarbonate for producing a pharmaceutical composition for treating or preventing nocturia due to nocturnal polyuria (e.g., idiopathic nocturnal polyuria);
(1-3) Use of citric acid or a pharmaceutically acceptable salt thereof, or hydrates thereof, or sodium bicarbonate for producing a pharmaceutical composition for treating or preventing nocturnal enuresis;
(1-4) Use of citric acid or a pharmaceutically acceptable salt thereof, or hydrates thereof, or sodium bicarbonate for producing a pharmaceutical composition for suppressing frequency of nocturnal urination;
(2-1) A pharmaceutical composition comprising citric acid or a pharmaceutically acceptable salt thereof, or hydrates thereof, or sodium bicarbonate for use in treating or preventing nocturnal polyuria;
(2-2) A pharmaceutical composition comprising citric acid or a pharmaceutically acceptable salt thereof, or hydrates thereof, or sodium bicarbonate for use in treating or preventing nocturia due to nocturnal polyuria (e.g., idiopathic nocturnal polyuria);
(2-3) A pharmaceutical composition comprising citric acid or a pharmaceutically acceptable salt thereof, or hydrates thereof, or sodium bicarbonate for use in treating or preventing nocturnal enuresis;
(2-4) A pharmaceutical composition comprising citric acid or a pharmaceutically acceptable salt thereof, or hydrates thereof, or sodium bicarbonate for use in suppressing frequency of nocturnal urination;
(3-1) A method for treating or preventing nocturnal polyuria, comprising administering an effective amount of a pharmaceutical composition comprising citric acid or a pharmaceutically acceptable salt thereof, or hydrates thereof, or sodium bicarbonate, to a subject in need of treatment or prevention for nocturnal polyuria;
(3-2) A method for treating or preventing nocturia due to nocturnal polyuria (e.g., idiopathic nocturnal polyuria), comprising administering an effective amount of a pharmaceutical composition comprising citric acid or a pharmaceutically acceptable salt thereof, or hydrates thereof, or sodium bicarbonate, to a subject in need of treatment or prevention for nocturia due to nocturnal polyuria (e.g., idiopathic nocturnal polyuria);
(3-3) A method for treating or preventing nocturnal enuresis, comprising administering an effective amount of a pharmaceutical composition comprising citric acid or a pharmaceutically acceptable salt thereof, or hydrates thereof, or sodium bicarbonate, to a subject in need of treatment or prevention for nocturnal enuresis;
(3-4) A method for suppressing frequency of nocturnal urination, comprising administering an effective amount of a pharmaceutical composition comprising citric acid or a pharmaceutically acceptable salt thereof, or hydrates thereof, or sodium bicarbonate, to a subject in need of suppression in frequency of nocturnal urination;
(4-1) The use, pharmaceutical composition, or method according to any of (1-1) to (1-4), (2-1) to (2-4), and (3-1) to (3-4), wherein the pharmaceutical composition is used in combination with a V2 receptor agonist;
(4-2) The use, pharmaceutical composition, or method according to any of (1-1) to (1-4), (2-1) to (2-4), (3-1) to (3-4), and (4-1), wherein the pharmaceutical composition is administered to a patient with chronic kidney disease;
(4-3) The use, pharmaceutical composition, or method according to any of (1-1) to (1-4), (2-1) to (2-4), (3-1) to (3-4), and (4-1) to (4-2), wherein a content of citric acid or a pharmaceutically acceptable salt thereof, or hydrates thereof in the pharmaceutical composition is an amount that improves acidic urine in gout or hyperuricemia;
(4-4) The use, pharmaceutical composition, or method according to any of (1-1) to (1-4), (2-1) to (2-4), (3-1) to (3-4), and (4-1) to (4-3), wherein citric acid or a pharmaceutically acceptable salt thereof, or hydrates thereof are sodium citrate or a hydrate thereof, potassium citrate or a hydrate thereof, or mixtures thereof;
(4-5) The use, pharmaceutical composition, or method according to any of (1-1) to (1-4), (2-1) to (2-4), (3-1) to (3-4), and (4-1) to (4-4) wherein citric acid or a pharmaceutically acceptable salt thereof, or hydrates thereof are sodium citrate or a hydrate thereof;
(4-6) The use, pharmaceutical composition, or method according to any of (1-1) to (1-4), (2-1) to (2-4), (3-1) to (3-4), and (4-1) to (4-5), wherein citric acid or a pharmaceutically acceptable salt thereof, or hydrates thereof are a mixture of sodium citrate dihydrate and potassium citrate monohydrate;
(4-7) The use, pharmaceutical composition, or method according to any of (1-1) to (1-4), (2-1) to (2-4), (3-1) to (3-4), and (4-1) to (4-6), wherein citric acid or a pharmaceutically acceptable salt thereof, or hydrates thereof are a mixture of sodium citrate dihydrate and potassium citrate monohydrate, and an oral dose of each of these ingredients is in a range of 0.5 g to 1.5 g/day for a total of 1 to 3 g/day;
(4-8) The use, pharmaceutical composition, or method according to any of (1-1) to (1-4), (2-1) to (2-4), (3-1) to (3-4), and (4-1) to (4-3), wherein citric acid or a pharmaceutically acceptable salt thereof, or hydrates thereof are a mixture of citric acid, sodium citrate dihydrate, and potassium citrate monohydrate;
(4-9) The use, pharmaceutical composition, or method according to any of (1-1) to (1-4), (2-1) to (2-4), (3-1) to (3-4), and (4-1) to (4-2), wherein a content of sodium bicarbonate in the pharmaceutical composition is an amount effective for improving acidosis, promoting uric acid excretion, or preventing gouty attacks;
(4-10) The use, pharmaceutical composition, or method according to any of (1-1) to (1-4), (2-1) to (2-4), (3-1) to (3-4), (4-1) to (4-2), and (4-9) wherein an oral dose of sodium bicarbonate is in a range of 3 g to 5 g/day;
(4-11) The use, pharmaceutical composition, or method according to any of (1-1) to (1-4), (2-1) to (2-4), (3-1) to (3-4), and (4-1) to (4-10), wherein the pharmaceutical composition is not administered to a patient with gout and hyperuricemia;
(4-12) The use, pharmaceutical composition, or method according to any of (1-1) to (1-4), (2-1) to (2-4), (3-1) to (3-4), and (4-1) to (4-11), wherein the pharmaceutical composition is administered for 1 week;
(4-13) The use, pharmaceutical composition, or method according to any of (1-1) to (1-4), (2-1) to (2-4), (3-1) to (3-4), and (4-1) to (4-12), wherein the pharmaceutical composition further comprises a V2 receptor agonist (e.g., desmopressin acetate hydrate);

The present invention also provides a food composition comprising citric acid, an edible salt of citric acid, or hydrates thereof, or mixtures thereof, or sodium bicarbonate.

In one embodiment, the food composition provided by the present invention is ingested by a healthy individual, which can exert beneficial effects (e.g., an effect of reducing nocturnal urine production, an effect of reducing the frequency of nocturnal urination, etc.).

In one embodiment, the food composition provided by the present invention does not affect the daily urine output.

In one embodiment, the food composition provided by the present invention does not affect the frequency of urination per day.

In one embodiment, the food composition provided by the present invention is ingested by an elderly individual (e.g., 60 years old or older, 65 years old or older, 70 years old or older, 75 years old or older, or 65 years old or older and under 75).

In one embodiment, the food composition provided by the present invention is administered to an individual 40 years old or older, 50 years old or older, or 40 years old or older and under 60.

In one embodiment, the food composition provided by the present invention is ingested by a young individual (e.g., an infant (e.g., a child 3 years old or older and under 6), a child (e.g., a child 6 years old or older), a child aged between 6 and 12, and a child aged between 6 and 15) .

In one embodiment, the food composition provided by the present invention is ingested by an age-conscious human.

In one embodiment, the food composition provided by the present invention is ingested by a human who is concerned about urge to urinate at night or urination at night (e.g., the frequency of nocturnal urination is high).

The content of citric acid, an edible salt of citric acid, or hydrates thereof, or mixtures thereof in the food composition provided by the present invention can be appropriately determined depending on the type of food. Examples of food compositions include foods for specified health use, nutritional supplements, functional foods, foods for hospital patients, and supplements. The form of these food compositions is not particularly limited as long as it contains an effective amount of citric acid, an edible salt of citric acid, or hydrates thereof, or mixtures thereof, or sodium bicarbonate in order to exert the above effect, and is orally ingestible. The food compositions may be in the form of a normal food or drink, or may be provided as a formulation suitable for oral administration, such as a tablet, a capsule, or a suspension, among the formulations used in the pharmaceutical composition. Known formulation technology can be applied to the constitution and production method of these formulations.

For example, in the case of foods for specified health use, nutritional supplements, functional foods or foods for hospital patients, anhydrous citric acid may be contained in an amount of 1/3 of 0.03 to 0.3 g, and potassium citrate monohydrate and sodium citrate dihydrate may be contained in a total amount of 1/3 of 1 to 3 g per serving. When foods for specified health use, nutritional supplements, functional foods, foods for hospital patients or supplements are provided as tablets, for example, 70 to 90% by weight of citric acid, an edible salt of citric acid, or hydrates thereof, or mixtures thereof may be contained in a tablet (300 mg to 600 mg). Further, when foods for specified health use, nutritional supplements, functional foods, foods for hospital patients or supplements are provided as tablets, for example, 70 to 90% by weight of sodium bicarbonate may be contained in a tablet (300 mg to 600 mg).

When the food composition provided by the present invention is not formulated and is provided in the form of a normal food or drink, it can be appropriately produced by those skilled in the art depending on the type of the food, for example, by blending a food material with citric acid, an edible salt of citric acid, or hydrates thereof, or mixtures thereof. Further, the food composition can be produced, for example, by blending a food material with sodium bicarbonate.

Examples of forms of the food or drink include liquid or milky or pasty foods such as beverage, soy sauce, milk, yogurt, and fermented soybean paste; semi-solid foods such as jelly and gummy candy; solid foods such as candy, gum, soybean curd, and supplement; and powdered foods.

Examples of beverages include fruit juice and fruit beverages, coffee beverages, oolong tea beverages, green tea beverages, black tea beverages, barley tea beverages, vegetable beverages, soft drinks such as carbonated beverages, fruit extract-containing beverages, vegetable extract-containing juices, flavored water, sports drinks, and diet drinks.

To beverages, additives such as antioxidants, fragrances, various esters, organic acids, organic acid salts, inorganic acids, inorganic acid salts, inorganic salts, pigments, emulsifiers, preservatives, seasoning agents, sweeteners, acidulants, fruit juice extracts, vegetable extracts, flower honey extracts, pH adjusters, and quality stabilizers can be added singly or in combination.

Further, examples of the embodiments provided by the present invention include the following:
(1-1) Use of citric acid or an edible salt thereof, or hydrates thereof, or sodium bicarbonate for producing a food product for reducing nocturnal urine production;
(1-2) Use of citric acid or an edible salt thereof, or hydrates thereof, or sodium bicarbonate for producing a food product for suppressing frequency of nocturnal urination;
(2-1) A food product comprising citric acid or an edible salt thereof, or hydrates thereof, or sodium bicarbonate for use in reducing nocturnal urine production;
(2-2) A food product comprising citric acid or an edible salt thereof, or hydrates thereof, or sodium bicarbonate for use in suppressing frequency of nocturnal urination;
(3-1) A method for reducing nocturnal urine production, comprising administering an effective amount of a food product comprising citric acid or an edible salt thereof, or hydrates thereof, or sodium bicarbonate to a subject in need of reduction of nocturnal urine production;
(3-2) A method for suppressing frequency of nocturnal urination, comprising administering an effective amount of a food product comprising citric acid or an edible salt thereof, or hydrates thereof, or sodium bicarbonate to a subject in need of suppression in frequency of nocturnal urination;
(4-1) The use, food product, or method according to any of (1-1), (1-2), (2-1), (2-2), (3-1), and (3-2), wherein citric acid or an edible salt thereof, or hydrates thereof are sodium citrate or a hydrate thereof, potassium citrate or a hydrate thereof, or mixtures thereof;
(4-2) The use, food product, or method according to any of (1-1), (1-2), (2-1), (2-2), (3-1), (3-2), and (4-1), wherein citric acid or an edible salt thereof, or hydrates thereof are sodium citrate or a hydrate thereof;
(4-3) The use, food product, or method according to any of (1-1), (1-2), (2-1), (2-2), (3-1), (3-2), (4-1), and (4-2), wherein citric acid or an edible salt thereof, or hydrates thereof are a mixture of sodium citrate dihydrate and potassium citrate monohydrate;
(4-4) The use, food product, or method according to any of (1-1), (1-2), (2-1), (2-2), (3-1), (3-2), and (4-1) to (4-3), wherein citric acid or an edible salt thereof, or hydrates thereof are a mixture of sodium citrate dihydrate and potassium citrate monohydrate, and an oral ingestion of each of these ingredients is in a range of 0.5 g to 1.5 g/day for a total of 1 to 3 g/day;
(4-5) The use, food product, or method according to any of (1-1), (1-2), (2-1), (2-2), (3-1), and (3-2), wherein citric acid or an edible salt thereof, or hydrates thereof are a mixture of citric acid, sodium citrate dihydrate, and potassium citrate monohydrate;
(4-6) The use, food product, or method according to any of (1-1), (1-2), (2-1), (2-2), (3-1), and (3-2), wherein an oral ingestion of sodium bicarbonate is in a range of 3 g to 5 g/day;
(4-7) The use, food product, or method according to any of (1-1), (1-2), (2-1), (2-2), (3-1), (3-2), and (4-1) to (4-6), wherein the food product is ingested by an elderly individual or young individual;
(4-8) The use, food product, or method according to any of (1-1), (1-2), (2-1), (2-2), (3-1), (3-2), and (4-1) to (4-7), wherein the food product is ingested by a human who is concerned about urge to urinate at night or urination at night; and
(4-9) The use, food product, or method according to any of (1-1), (1-2), (2-1), (2-2), (3-1), (3-2), and (4-1) to (4-8), wherein the food product is ingested by an age-conscious human.

Hereinafter, the present invention will be further described with reference to Examples, but the present invention is not limited thereto.

### Examples

An open-label crossover test for aiming at confirming the effects of a combination formulation of potassium citrate, sodium citrate hydrate, and citric acid as well as a formulation of sodium bicarbonate (baking soda) on nocturnal urine output, frequency of nocturnal urination, and urine pH was performed on healthy males (aged 29 to 63 years) (the number of entries: 30). The subjects were given the following procedures 1) to 3) at intervals of 1 week or longer:
1) oral administration of two tablets each containing 231.5 mg of potassium citrate (C₆H₅K₃O₇·H₂O), 195.0 mg of sodium citrate hydrate (C₆H₅Na₃O₇·2H₂O), and 72.5 mg of anhydrous citric acid three times per day (morning, noon, evening) for 7 days (Group A: citric acid formulation administration group);
2) oral administration of four tablets each containing 500 mg of sodium bicarbonate three times per day (morning, noon, evening) for 7 days (Group B: baking soda administration group); and
3) no drug administration for 7 days (Group C: control).

On the last day of drug administration in each group, urine was collected for 24 hours using Urinemate (registered trademark) P (obtained from Sumitomo Bakelite Co., Ltd.), and the time, urine output, and urine pH were recorded for each urination. The "urine output between 22:00 (after) and early morning first urine" was defined as "nocturnal urine" and the "urine output between second urine and 22:00" was defined as "diurnal urine".

Regarding the "nocturnal urine" and the "diurnal urine", the "urine output" and the "frequency of urination" were compared among the three groups. In addition, the relationship between the effects of "first urine" and "second urine" on pH and urine output was investigated.

On the urine collection day, the diet of the subjects was controlled, and the influence of diet (ingestion dose of salt, sugars, and protein, etc.) among the subjects was eliminated as much as possible.

The Wilcoxon signed rank test was used for statistical analysis.

The results are shown in Tables 1 to 3.

**[Table1]**

| Influence of administration of citric acid formulation and baking soda formulation on nocturnal urine volume | | | | | | | |
|---|---|---|---|---|---|---|---|
| Group | No. | Diurnal urine (mL) | Nocturnal urine (mL) | 24-hour urine (mL) | Nocturnal urine/24-hour urine (%) | Diurnal urine-nocturnal urine (mL) | P value (diurnal vs nocturnal) |
| Control (C) | 27 | 991±417 | 554±370 | 1545±571 | 36±15 | 438±544 | 0.0004 |
| Citric acid formulation (A) | 29 | 1024±345 | 421±184 | 1445±384 | 30±11 | 603±397 | < 0.0001 |
| Baking soda formulation (B) | 26 | 1106±392 | 499±183 | 1605±343 | 33±13 | 607±506 | < 0.0001 |
| P value (vs C) | | 0.3011 (A) | 0.0999 (A) | 0.6319 (A) | 0.0126 (A) | 0.0484 (A) | |
| | | 0.0814 (B) | 0.9944 (B) | 0.0814 (B) | 0.2382 (B) | 0.1283 (B) | |
| Mean + SD | | | | | | | |

**[Table 2]**

| Influence of administration of citric acid formulation and baking soda formulation on frequency of urination | | | | | |
|---|---|---|---|---|---|
| Group | No. | Diurnal (frequency) | Nocturnal (frequency) | 24-hour urine (frequency) | P value (diurnal vs nocturnal) |
| Control (C) | 27 | 4.1±1.2 | 2.2±1.1 | 6.3±1.7 | < 0.0001 |
| Citric acid formulation (A) | 29 | 4.1±1.0 | 1.9±0.7 | 6.0±1.2 | < 0.0001 |
| Baking soda formulation (B) | 26 | 4.2±1.4 | 1.8±0.6 | 6.0±1.5 | < 0.0001 |
| P value (vs C) | | 0.8160 (A) | 0.1309 (A) | 0.5024 (A) | |
| | | 0.8748 (B) | 0.0898 (B) | 0.4316 (B) | |
| Mean±SD | | | | | |

**[Table 3]**

| Influence of administration of citric acid formulation and baking soda formulation on urine pH | | | | | | |
|---|---|---|---|---|---|---|
| Group | No. | Early morning first urine | | Early morning second urine | | P value (first urine vs second urine) |
| | | pH | ΔpH | pH | ΔpH | |
| Control (C) | 27 | 5.87±0.54 | - | 6.28±0.61 | - | 0.0055 |
| Citric acid formulation (A) | 29 | 6.31±0.58 | 0.49±0.69 | 6.91 ±0.51 | 0.61±0.64 | 0.0007 |
| Baking soda formulation (B) | 26 | 6.79±0.61 | 0.94±0.67 | 7.19±0.42 | 0.95±0.69 | 0.0055 |
| P value | | 0.0007 (A vs C) | | < 0.0001 (A vs C) | | |
| | | < 0.0001 (B vs C) | 0.0012 | < 0.0001 (B vs C) | 0.0003 | |
| | | 0.0013 (A vs B) | | 0.0006 (A vs B) | | |
| Mean±SD | | | | | | |

As a result, the administration of the citric acid formulation (Group A) reduced the nocturnal urine output compared to a control group (Group C) (Table 1). Further, the administration of the citric acid formulation (Group A) reduced the percentage of nocturnal urine output in the daily urine output compared to the control group (Group C) (Table 1). The administration of the baking soda formulation (Group B) also reduced the nocturnal urine output compared to the control group (Group C) (Table 1). Furthermore, the administration of the baking soda formulation (Group B) reduced the percentage of nocturnal urine output in the daily urine output compared to the control group (Group C) (Table 1).

Regarding the frequency of urination, the administration of the citric acid formulation (Group A) did not change the frequency of diurnal urination, compared to the control group (Group C), but the frequency of nocturnal urination was decreased (Table 2). The administration of the baking soda formulation (Group B) did not change the frequency of diurnal urination, compared to the control group (Group C), but the frequency of nocturnal urination was decreased (Table 2).

The urine pH after administration of the citric acid formulation (Group A) and the urine pH after administration of the baking soda formulation (Group B) were significantly increased (alkalized), compared to the urine pH in the control group (Group C). As for the tested dose, the urine pH after administration of the baking soda formulation (Group B) was increased (alkalized), compared to the urine pH after administration of the citric acid formulation (Group A) (Table 3). However, the degree of urinary alkalinization effect of the citric acid formulation and the baking soda formulation was not reflected in the degree of the nocturnal urine output-reducing effect of both the formulations. Consequently, it was suggested that the effect of the citric acid formulation on reducing the nocturnal urine output was due not only to the urinary alkalinization but also to the effect of citric acid other than the urinary alkalinization effect of the citric acid formulation (Tables 1 and 3). Similarly, the degree of urinary alkalinization effect of the citric acid formulation and the baking soda formulation was not reflected in the degree of the nocturnal urination frequency-reducing effect of both the formulations. Therefore, it was suggested that the effect of the citric acid formulation on reducing the frequency of nocturnal urination was due not only to the urinary alkalinization but also to the effect of citric acid other than the urinary alkalinization effect of the citric acid formulation (Tables 2 and 3).

### Industrial Applicability

The prevention or treatment of nocturnal polyuria, decrease in frequency of nocturnal urination, prevention or treatment of nocturnal enuresis, and the like in mammals can be achieved by administering the pharmaceutical composition provided by the present invention, or the like.

## Claims

1. A therapeutic or prophylactic agent for nocturnal polyuria, wherein the therapeutic or prophylactic agent is a tablet comprising citric acid, a pharmaceutically acceptable salt of citric acid, or hydrates thereof, or mixtures thereof.

2. A therapeutic or prophylactic agent for nocturnal enuresis, wherein the therapeutic or prophylactic agent is a tablet comprising sodium citrate or a hydrate thereof, potassium citrate or a hydrate thereof, or mixtures thereof.

3. The therapeutic or prophylactic agent according to claim 1 or 2, wherein the therapeutic or prophylactic agent is a medicine.

4. The therapeutic or prophylactic agent according to any one of claims 1 to 3, wherein the therapeutic or prophylactic agent is used in combination with a V2 receptor agonist.

5. The therapeutic or prophylactic agent according to any one of claims 1 to 4, wherein the therapeutic or prophylactic agent is designed so that citric acid or a pharmaceutically acceptable salt thereof, or hydrates thereof are administered in an amount that improves acidic urine in gout or hyperuricemia.

6. The therapeutic or prophylactic agent according to any one of claims 1 to 5, wherein the therapeutic or prophylactic agent is administered to a patient with chronic kidney disease.

7. An agent for suppressing frequency of nocturnal urination, comprising citric acid, a pharmaceutically acceptable salt of citric acid, or hydrates thereof, or mixtures thereof, wherein the agent is a tablet.

8. The agent according to claim 7, wherein the agent is a medicine or food.

9. The therapeutic or prophylactic agent, or the agent for suppressing frequency of nocturnal urination according to any one of claims 1 to 8, wherein the citric acid or a pharmaceutically acceptable salt thereof, or hydrates thereof are sodium citrate or a hydrate thereof, potassium citrate or a hydrate thereof, or mixtures thereof.

10. The therapeutic or prophylactic agent, or the agent for suppressing frequency of nocturnal urination according to any one of claims 1 to 9, wherein the citric acid or a pharmaceutically acceptable salt thereof, or hydrates thereof are sodium citrate or a hydrate thereof.

11. The therapeutic or prophylactic agent, or the agent for suppressing frequency of nocturnal urination according to any one of claims 1 to 10, wherein the citric acid or a pharmaceutically acceptable salt thereof, or hydrates thereof are a mixture of sodium citrate dihydrate and potassium citrate monohydrate.

12. The therapeutic or prophylactic agent, or the agent for suppressing frequency of nocturnal urination according to any one of claims 1 to 11, wherein the citric acid or a pharmaceutically acceptable salt thereof, or hydrates thereof are a mixture of sodium citrate dihydrate and potassium citrate monohydrate, and an oral dose or ingestion dose of each ingredient is in a range of 0.5 g to 1.5 g/day for a total of 1 to 3 g/day.

13. A therapeutic or prophylactic agent for nocturnal polyuria, wherein the therapeutic or prophylactic agent is a tablet comprising sodium bicarbonate.

14. A therapeutic or prophylactic agent for nocturnal enuresis, wherein the therapeutic or prophylactic agent is a tablet comprising sodium bicarbonate.

15. The therapeutic or prophylactic agent according to claim 13 or 14, wherein the therapeutic or prophylactic agent is a medicine.

16. The therapeutic or prophylactic agent according to any one of claims 13 to 15, wherein the therapeutic or prophylactic agent is used in combination with a V2 receptor agonist.

17. The therapeutic or prophylactic agent according to any one of claims 13 to 16, wherein the therapeutic or prophylactic agent is designed so that sodium bicarbonate is administered in an amount effective for improving acidosis, promoting uric acid excretion, or preventing gouty attacks.

18. The therapeutic or prophylactic agent according to any one of claims 13 to 17, wherein the therapeutic or prophylactic agent is administered to a patient with chronic kidney disease.

19. An agent for suppressing frequency of nocturnal urination, wherein the agent is a tablet comprising sodium bicarbonate.

20. The agent according to claim 19, wherein the agent is a medicine or food.

21. The therapeutic or prophylactic agent, or the agent for suppressing frequency of nocturnal urination according to any one of claims 13 to 20, wherein an oral dose or ingestion dose of sodium bicarbonate is in a range of 3 g to 5 g/day.

22. The therapeutic or prophylactic agent, or the agent for suppressing frequency of nocturnal urination according to any one of claims 1 to 21, wherein the agent is administered for 1 week.
